# EUROPEAN PATENT SPECIFICATION

(11) **EP 1 347 300 B1**
(45) Date of publication and mention of the grant of the patent: **18.04.2007**
(21) Application number: 01998196.8
(22) Date of filing: 30.11.2001
(51) Int. Cl.: G01N 33/543, G01N 33/557, G01N 33/68

(54) **METHOD OF MEASURING BINDING ACTIVITY OF LIGAND-BINDING PROTEIN HAVING POOR CHEMICAL STABILITY TO FIRST LIGAND**
MESSVERFAHREN FÜR DIE BINDUNGSAKTIVITÄT EINES LIGANDEN BINDENDEN PROTEINS MIT GERINGER CHEMISCHER STABILITÄT FÜR DEN ERSTEN LIGANDEN
PROCEDE DE MESURE DE L'ACTIVITE DE LIAISON D'UNE PROTEINE SE LIANT A UN LIGAND PRESENTANT UNE FAIBLE STABILITE CHIMIQUE A UN PREMIER LIGAND

(30) Priority: 30.11.2000 JP 2000364593
(43) Date of publication of application: 24.09.2003
(73) Proprietor: CHUGAI SEIYAKU KABUSHIKI KAISHA, Tokyo, 115-8543 (JP)
(72) Inventor: Kakuta, Masaya c/o Chugai Seiyaku Kabushiki K., Tokyo 171-8545 (JP); Tsuchiya, Mikako c/o Chugai Seiyaku Kabushiki K., Tokyo 171-8545 (JP)
(74) Representative: Vossius & Partner
(86) International application number: PCT/JP2001/010494
(87) International publication number: WO 2002/044727

(56) References cited:
- EP-A- 0 722 088
- EP-A- 1 296 140
- JP-A- 4 506 563
- JP-A- 8 319 300
- JP-A- 63 271 162
- CATIMEL B ET AL: "Kinetic analysis of the interaction between the monoclonal antibody A33 and its colonic epithelial antigen by the use of an optical biosensor - A comparison of immobilisation strategies" JOURNAL OF CHROMATOGRAPHY A, ELSEVIER, AMSTERDAM, NL, vol. 776, no. 1, 25 July 1997 (1997-07-25), pages 15-30, XP004125534 ISSN: 0021-9673
- MONTERO-JULIAN F A ET AL: "Immunoassay for functional human soluble interleukin-6 receptor in plasma based on ligand/receptor interactions" JOURNAL OF IMMUNOLOGICAL METHODS, vol. 169, no. 1, 1994, pages 111-121, XP002327354 ISSN: 0022-1759
- WARD LARRY D ET AL: "Use of a Biosensor with Surface Plasmon Resonance Detection for the Determination of Binding Constants: Measurement of Interleukin-6 Binding to the Soluble interleukin-6 Receptor" BIOCHEMISTRY, vol. 34, no. 9, 1995, pages 2901-2907, XP002327355 ISSN: 0006-2960
- CANZIANI G ET AL: "Exploring Biomolecular Recognition Using Optical Biosensors" METHODS : A COMPANION TO METHODS IN ENZYMOLOGY, ACADEMIC PRESS INC., NEW YORK, NY, US, vol. 19, no. 2, October 1999 (1999-10), pages 253-269, XP004466829 ISSN: 1046-2023
- DECKERT F. ET AL.: 'Development and validation of an IL-6 immuno-receptor assay based on sueface plasmon resonance' J. PHARM. BIOMED. ANAL. vol. 23, no. 2-3, 15 August 2000, pages 403 - 412, XP002907646
- TAREMI S.S. ET AL.: 'Human interleukin 4 receptor complex: neutralization effect of two monoclonal antibodies' BIOCHEMISTRY vol. 35, no. 7, 20 February 1996, pages 2322 - 2331, XP002907647

## Description

### TECHNICAL FIELD

The present invention relates to methods for assaying the binding activity of a ligand to a chemically less stable ligand-binding protein.

### BACKGROUND ART

Various biological reactions are induced and controlled by specific interactions between molecules constituting living bodies. For example, biological reactions are controlled by specific association/dissociation reactions between biomolecules such as enzyme-substrate reactions, antigen-antibody reactions, binding of a receptor to its ligand or formation of a transcription factor-DNA complex. To understand such biological reactions at the molecular level, analyses of molecular interactions are needed.

For example, conventional assays include radioimmunoassays (RIA) and enzyme-linked immunosorbent assays (ELISA) for interactions between antibodies and antigens; filter binding assays for binding between receptors and their ligands; and gel shift assays for binding between transcription factors and DNAs. All of these assays comprise labeling one molecule with a radioisotope or a fluorescent dye and measuring the amount of the complex once the binding reaction reaches equilibrium.

Recently, it has become possible to monitor molecular interactions in real time without labeling by applying an optical phenomenon called surface plasmon resonance (hereinafter referred to as "SPR"). For example, equipment for analyzing molecular interactions using a surface plasmon resonance sensor is commercially available under the trade name BIACORE (from Pharmacia Biotech, currently BIACORE). Molecular interactions can also be monitored without labeling by applying an optical phenomenon called mirror resonance using equipments commercially available under the trade name IAsys (Affinity sensors).

BIACORE systems basically consist of a light source, a prism, a detector and a microflow channel. In practice, one of interactant molecules is immobilized on a cassette-type sensor chip and a sample containing a molecule acting on it is injected via a cassette-type microflow channel system to optically detect minor mass changes on the surface of the sensor chip due to the association or dissociation between the two molecules.

The principle of such detection systems is based on a phenomenon called surface plasmon resonance. That is, the light incident to be totally reflected on the interface between glass and a metal thin film is partially used to excite surface plasmons and attenuated at a certain angle of incidence. This angle varies with the concentration of the solvent in contact with the metal thin film (sensor). SPR detects this variation.

In BIACORE systems, this variation is called resonance signal (SPR signal) and expressed in RU (resonance units). A response of 1,000 RU corresponds to a variation of 0.1 degree, or a variation when about 1 ng of proteins binds on a thin metal sensor having a surface area of 1 mm². These systems can sufficiently detect changes of about 50 RU (50 pg) of proteins.

The detected signal is converted into a binding curve called a sensorgram by a computer contained in SPR systems and plotted in real time on a computer display (Natsume, T. et al., (1995) Experimental Medicine 13, pp. 563-569.) (Karlsson, R. et al., (1991) J. Immunol. Methods 145, pp. 229-240.).

Interactions between an antibody and an antigen are determined by SPR as follows. When a sample containing an antibody recognizing an antigen is injected onto a sensor chip on which the antigen is immobilized, for example, the mass of the surface of the sensor chip increases by a specific antigen-antibody reaction and the refractive index on the surface of the sensor chip also increases. Association and dissociation between the antigen and the antibody can be detected by measuring the angle at which the reflected light disappears by changes in refractive index (SPR angle). In such an assay, the antibody bound is normally washed off after a measurement and the antigen immobilized on the sensor chip is reused.

If the antigen immobilized on the sensor chip is a chemically less stable protein, however, it cannot be reused because it is deactivated by washing.

Such chemically less stable proteins include IL-6 receptors that are antigens recognized by anti-IL-6 receptor antibodies. Anti-IL-6 receptor antibodies have been found to show a therapeutic effect, by blocking IL-6 signal transduction in immature myeloma cells to inhibit biological activity of IL-6, for various diseases in which IL-6 is involved such as immunopathies, inflammatory diseases and lymphoma (Tsunenari, T. et al., Blood, 90: 2437, 1997; Tsunenari, T. et al., Anticancer Res. 16: 2537, 1996).

Biological activity tests of anti-IL-6 receptor antibodies have been performed by applying ELISA based on the binding of an anti-IL-6 receptor antibody to its antigen IL-6 receptor but with an accuracy of 10-20% in CV value (Coefficient of variation). On the other hand, it would be desirable to establish a biological activity assay with higher accuracy in order to evaluate in detail the binding activity of electrostatically heterogenic molecules or oligomeric products of anti-IL-6 receptor antibodies. In these circumstances, we tried to develop an SPR-based biological activity assay with high reproducibility and high accuracy. A problem here was that IL-6 receptors are chemically less stable proteins that cannot be reused on the sensor chip surface as described above.

Thus, it is necessary to provide a method that allows the biological activity of an antibody to be simply and accurately determined while maintaining the specificity of SPR even when a chemically less stable antigen is used.

The present invention aims to provide a simple and accurate biological activity assay for ligands (especially antibodies).

### DISCLOSURE OF THE INVENTION

We studied to develop an SPR-based method for evaluating the biological activity of an antibody even against a chemically less stable antigenic protein wherein a sensor chip can reuse repetitively. As a result, we succeeded to establish an accurate and simple method for evaluating the activity of an antibody by immobilizing a second antibody on a sensor chip to allow the second antibody to bind to an antigen and then measuring the binding activity of the antibody to be assayed for biological activity to the antigen via surface plasmon resonance, and finally we accomplished the present invention.

Accordingly, the present invention provides a method for assaying the binding activity of a first ligand to a chemically less stable ligand-binding protein, comprising immobilizing a second ligand that binds to the ligand-binding protein at a binding site distinct from the binding site of the first ligand to the ligand-binding protein, binding the protein to the second ligand, and then measuring the binding activity of the first ligand to the protein via surface plasmon resonance.

The present invention also provides a method for assaying the biological activity of a first ligand that binds to a chemically less stable ligand-binding protein, comprising immobilizing a second ligand that binds to the ligand-binding protein at a binding site distinct from the binding site of the first ligand to the ligand-binding protein, binding the protein to the second ligand, and then determining the biological activity of the first ligand by measuring the binding activity of the first ligand to the protein via surface plasmon resonance.

The present invention also provides a kit for assaying the biological activity of a first ligand that binds to a chemically less stable ligand-binding protein via surface plasmon resonance, comprising (1) the ligand-binding protein and (2) a second ligand that binds to the ligand-binding protein at a binding site distinct from the binding site of the first ligand to the ligand-binding protein.

### BRIEF DESCRIPTION OF DRAWINGS

Fig. 1 shows a schematic view of a system for evaluating the binding activity of an hPM-1 antibody (humanized anti-IL-6R antibody) to IL-6R.
Fig. 2 shows a sensorgram in measuring the preconcentration of MT-18 (mouse anti-human IL-6R antibody).
Fig. 3 shows an example of a sensorgram in which MT-18 is immobilized.
Fig. 4 is a sensorgram showing that sIL-6R binds to MT-18 immobilized on a sensor chip and that hPM-1 binds to SIL-6R.

### PREFERRED EMBODIMENTS OF THE INVENTION

The chemically less stable ligand-binding protein means a protein that specifically binds to its ligands and that has less chemical stability. Specifically, it means a protein having a ligand-binding activity reduced to 80% or less, particularly 50% or less after one cycle of washing (for example, washing with an acidic solution such as 10 mM Gly-HCl (pH 2) or 10-100 mM HCl, an alkaline solution such as 10 mM Gly-NaOH (pH 11) or 10-100 mM NaOH, a high-salt solution such as 1-5 M NaCl, or a protein denaturant such as 0.5% SDS or 8 M Guanidine-HCl) on a sensor chip to which it is immobilized. The chemically less stable ligand-binding protein may be, for example, a chemically less stable antigenic protein, receptor protein, enzyme or transcription factor. Particularly, the chemically less stable ligand-binding protein is an antigenic protein. Especially, the methods of the present invention are suitably applied to determine the ligand-binding activity of proteins solubilized, such as soluble receptors, that normally bind to cell membranes or the like and that have a relatively high molecular weight and complicated structure, because the binding sites of the proteins to their ligands such as antibodies are less stable. The soluble receptors may be receptors for peptide hormones or cytokines, particularly cytokine receptors. Examples of the cytokine receptors include receptors for growth factors, lymphokines, monokines, interleukins, interferons, chemokines, colony-stimulating factors, hematopoietic factors, neurotrophic factors and differentiation-inhibiting factors. More specifically, erythropoietin (EPO) receptors, thrombopoietin (TPO) receptors, granulocyte colony-stimulating factor (G-CSF) receptors, macrophage colony-stimulating factor (M-CSF) receptors, granulocyte macrophage colony-stimulating factor (GM-CSF) receptors, tumor necrosis factor (TNF) receptors, interleukin-1 (IL-1) receptors, interleukin-2 (IL-2) receptors, interleukin-3 (IL-3) receptors, interleukin-4 (IL-4) receptors, interleukin-5 (IL-5) receptors, interleukin-6 (IL-6) receptors, interleukin-7 (IL-7) receptors, interleukin-9 (IL-9) receptors, interleukin-10 (IL-10) receptors, interleukin-11 (IL-11) receptors, interleukin-12 (IL-12) receptors, interleukin-13 (IL-13) receptors, interleukin-15 (IL-15) receptors, interferon-α (IFN-α) receptors, interferon-β (IFN-β) receptors, interferon-γ (IFN-γ) receptors, growth hormone (GH) receptors, insulin receptors, stem cell factor (SCF) receptors, vascular endothelial growth factor (VEGF) receptors, epidermal growth factor (EGF) receptors, nerve growth factor (NGF) receptors, fibroblast growth factor (FGF) receptors, platelet-derived growth factor (PDGF) receptors, transforming growth factor-β (TGF-β) receptors, leukocyte migration inhibitory factor (LIF) receptors, ciliary neurotrophic factor (CNTF) receptors, oncostatin M (OSM) receptors and Notch family receptors are exemplified. Especially preferred are IL-6 receptors. Accordingly, especially preferred chemically less stable ligand-binding proteins are soluble IL-6 receptors (Taga, Cell 58: 573-581, 1989; Yasukawa, J. Biochem. 108: 673-679, 1990).

The first ligand is a ligand that specifically binds to the chemically less stable ligand-binding protein. When the chemically less stable ligand-binding protein is e.g. an antigenic protein, the first ligand may be an antibody, an antibody fragment such as Fab or F(ab)₂, or a single-chain antibody Fv either of which recognizes the protein. When the chemically less stable ligand-binding protein is a soluble IL-6 receptor, the first ligand is preferably an anti-IL-6 receptor antibody, especially a humanized anti-IL-6 receptor antibody.

Alternatively, when the chemically less stable ligand-binding protein is a receptor protein, the first ligand may be a ligand for the receptor protein. When the ligand-binding protein is e.g. an IL-6 receptor (IL-6R), the first ligand is IL-6. In this case, the methods of the present invention can be applied to evaluate the quality of IL-6 or to determine IL-6 levels in blood by measuring the binding activity of IL-6 to IL-6R. When the chemically less stable ligand-binding protein is an enzyme, the first ligand may be a substrate for the enzyme, and when the chemically less stable ligand-binding protein is a transcription factor, the first ligand may be a DNA that interacts with the transcription factor.

Alternatively, the first ligand may be a synthetic compound or peptide having an agonistic or antagonistic effect on the ligand-binding protein.

The second ligand is a ligand that binds to the chemically less stable ligand-binding protein at the binding site distinct from the binding site of the first ligand to the ligand-binding protein. When the chemically less stable ligand-binding protein is an antigenic protein, the second ligand may be an antibody that recognizes a different epitope from that of the first ligand, i.e. another antibody against the antigenic protein. When the chemically less stable ligand-binding protein is an IL-6 receptor, a mouse anti-human IL-6 receptor antibody or the like can be used as the second ligand. For example, MT-18 (Hirata et al., J. Immunol. 143: 2900-2906, 1989) can be used as a mouse anti-human IL-6 receptor antibody.

The antibody used as the first or second ligand may be a polyclonal antibody or a monoclonal antibody, particularly a monoclonal antibody. The class of the antibody may be any of IgM, IgD, IgG, IgA or IgE. Antibody fragments such as Fab and (Fab')₂, and reshaped antibodies such as monovalent or multivalent single-chain antibodies (scFv) are also included. Desirably, the antibody forms no oligomeres such as a dimer, or the antibody forms little oligomers (preferably 5% or less).

These antibodies against a protein can be prepared by, for example, purifying the protein from cells or culture media expressing the protein or a recombinant thereof and immunizing a rabbit or the like with the purified protein combined with a suitable adjuvant to obtain an antibody fraction from the serum according to standard methods. Alternatively, monoclonal antibodies can be prepared using mice or rats, or humanized antibodies or single-chain antibodies can be prepared using gene recombination techniques or transgenic animals, though the present invention is not limited to these specific methods.

In preferred embodiments of the present invention, the chemically less stable ligand-binding protein is an antigenic protein, the first ligand is a first antibody recognizing the antigenic protein, and the second ligand is a second antibody recognizing an epitope distinct from that of the first antibody.

In an especially preferred embodiment of the present invention, the chemically less stable ligand-binding protein is an IL-6 receptor, the first ligand is a humanized anti-IL-6 receptor antibody, and the second ligand is a mouse anti-human IL-6 receptor antibody.

In a method according to the especially preferred embodiment of the present invention, the anti-IL-6 receptor antibody used as the first ligand can be obtained by using the nucleotide sequence of human IL-6R disclosed in e.g. European Patent Application Publication No. EP325474 as a sensitizing antigen. That is, the antibody can be obtained by the following known method. The nucleotide sequence of human IL-6R is inserted into a known expression vector system to transform a suitable host cell, and then the desired IL-6R protein is purified from the host cell or culture supernatant thereof and this purified IL-6R protein is used as a sensitizing antigen. In the present invention, reshaped humanized antibodies can be used. Such antibodies are obtained by replacing the complementarity determining regions of a human antibody by the complementarity determining regions of a non-human mammalian antibody such as a mouse antibody by general gene recombination techniques which are also known. By using these known techniques reshaped humanized antibodies useful for the present invention can be obtained. If necessary, reshaped humanized antibodies may have some amino acid substitutions in the framework regions (FRs) of the variable regions so that the complementarity determining regions form an appropriate antigen-binding site (Sato et al., Cancer Res. 53: 1-6, 1993). Such reshaped humanized antibodies are preferably exemplified by humanized PM-1 antibodies (hPM-1) (see International Publication No. WO92/19759).

In order to assay the binding activity of a first ligand to a chemically less stable ligand-binding protein via surface plasmon resonance (SPR) according to the present invention, a second ligand is prepared in a suitable buffer (for example, Na-acetate buffer) and injected and immobilized on a sensor chip. To efficiently immobilize the ligand on the sensor chip, the type, pH and flow rate of the buffer in which the ligand is concentrated on the sensor chip are appropriately chosen. In order to more accurately determine the concentration via SPR, the ligand is preferably immobilized in large quantities (about several thousands to ten thousands of RU) (Kazuhiro Nagata et al. ed. "Experimental Procedures for Real-Time Analysis of Biomolecular interaction", Springer-Verlag Tokyo, 1998, p.42).

Then, the ligand-binding protein prepared in a suitable buffer is injected so that the ligand-binding protein binds to the second ligand. Then, the first ligand prepared in a suitable buffer is also injected to measure the binding activity between the ligand-binding protein and the first ligand.

The binding activity can be measured and evaluated by plotting a sensorgram using an SPR system such as BIACORE 2000 (BIACORE) and calculating the activity from the sensorgram using commercially available software (for example, BIAevaluation Ver. 3.0: BIACORE).

After the measurement, a regeneration solution is injected to dissociate the ligand and the protein and the sensor chip is washed. Generally, a solution with varying salt concentrations or pHs (for example, an acidic solution such as 10 mM Gly-HCl (pH 2) or 10-100 mM HCl, an alkaline solution such as 10 mM Gly-NaOH (pH 11) or 10-100 mM NaOH, a high-salt solution such as 1-5 M NaCl, or a protein denaturant such as 0.5% SDS or 8 M Guanidine-HCl) is used as the regeneration solution. If regeneration is insufficient, the performance of the sensor chip is lowered because the protein is not completely dissociated from the ligand. Thus, the sensor chip should be sufficiently washed by appropriately selecting regeneration conditions such as the type and flow rate of the regeneration solution and the amount to be injected.

The present invention also provides a method for assaying the biological activity of a first ligand that binds to a chemically less stable ligand-binding protein, comprising immobilizing a second ligand that binds to the ligand-binding protein at a binding site distinct from the binding site of the first ligand to the ligand-binding protein, binding the protein to the second ligand, and then determining the biological activity of the first ligand by measuring the binding activity of the first ligand to the protein via surface plasmon resonance. Thus, the biological activity of the first ligand can be obtained with high efficiency and high accuracy.

The present invention also provides a kit for assaying the biological activity of a first ligand that binds to a chemically less stable ligand-binding protein via surface plasmon resonance, comprising (1) the ligand-binding protein and (2) a second ligand that binds to the ligand-binding protein at a binding site distinct from the binding site of the first ligand to the ligand-binding protein.

Surface plasmon resonance-based assays of the present invention are convenient because interactions between biomolecules, e.g. interactions between an antibody and an antigen can be monitored in real time without labeling. Once the second ligand is immobilized on the sensor chip, the ligand can be repetitively reused to test a number of samples. As shown in Examples below, surface plasmon resonance-based assays of the present invention showed higher accuracy than ELISA-based assays.

The method for assaying binding activity and the method and kit for assaying the biological activity of a first ligand (for example, an antibody) according to the present invention allow the relative binding activity of the ligand (for example, antibody) molecules to be evaluated without adding any purification step even if foreign proteins exist as impurities or culture media or stabilizers in sample solutions. Thus, the methods and kit of the present invention can be applied to quality control or in-process control of antibodies in the preparation process or as purified stocks, or to formulation design studies.

The methods and kit of the present invention can also be applied as methods for screening a ligand, such as an antibody or a variant of a natural ligand, having a high binding activity for a ligand-binding protein such as a receptor. For example, the methods and kit of the present invention are useful for selecting a monoclonal antibody, an antibody fragment, a single-chain antibody or the like each of which has a high binding activity for an antigenic protein.

The methods and kit of the present invention are also useful as methods for screening a synthetic compound or a peptide each of which has an agonistic or antagonistic effect on a ligand-binding protein such as a receptor.

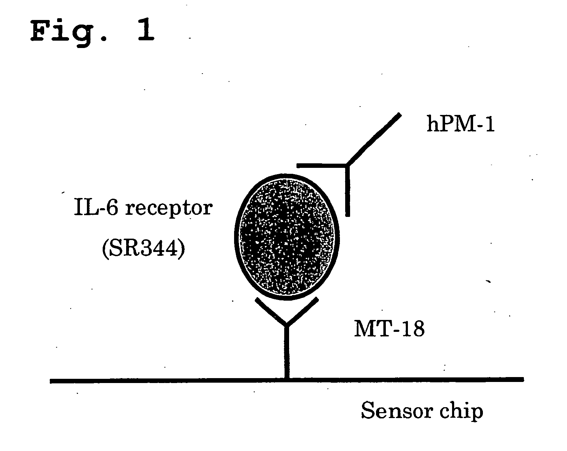

As an example of a method of the present invention, an SPR-based system for evaluating the binding activity of an hPM-1 antibody (humanized anti-IL-6R antibody) to sIL-6R (soluble human IL-6R) by binding the sIL-6R to MT-18 (mouse anti-human IL-6R antibody) immobilized as a ligand on a sensor chip and then allowing the hPM-1 antibody to bind to the sIL-6R is explained below. A schematic view of this system is shown in Fig. 1.

### INDUSTRIAL APPLICABILITY

According to the present invention, a method for assaying the binding activity of even a chemically less stable protein to a ligand and a method for assaying the biological activity of a ligand were provided. The methods of the present invention are convenient and highly accurate.

### EXAMPLES

The following Examples further illustrate the present invention. These Examples are given for illustrative purposes only and should not construed to limit the scope of the present invention.

Samples, reagents and equipments used in the following Examples are shown below.

### Samples

·hPM-1 antibody (humanized anti-IL-6R antibody) stock

### Reagents

**·** Amine-coupling kit (BIACORE)
   N-Ethyl-N'-(3-dimethyl-amino-propyl)-carbodiimide hydrochloride (EDC)
   N-Hydrosuccinimide (NHS)
   1 M Ethanolamine-HCl pH 8.5
·HBS-EP buffer (BIACORE) (0.01 M HEPES pH 7.4, 0.15 M Sodium chloride, 3 mM EDTA, 0.005% Surfactant P20)
·MT-18 (mouse anti-human IL-6 receptor antibody) (prepared according to the literature of Hirata et al., supra.) ·Soluble human IL-6 receptor (prepared according to the literature of Yasukawa, supra.)
·Alkaline phosphatase-labeled anti-human IgG antibody (BIOSOURCE)
·SIGMA 104 (SIGMA)

### Instruments and equipment

·BIACORE 2000 (BIACORE)
·SPECTRA max (Molecular Devices)
·Microplate Washer EW-812 (Molecular Devices)
·SOFT max (Molecular Devices)
·Sensor chip CM5 Research grade (BIACORE)
·F96 Cert. maxisorp NUNC-ImmunoPlate (Intermed)

### Data analysis method and software

·BIAevaluation Ver. 3.0 (BIACORE)

### Example 1: Preconcentration of MT-18

A means for efficiently immobilizing a ligand on a sensor chip is to find suitable conditions under which the ligand is concentrated on the sensor chip. For SPR assays, the ligand is concentrated on the sensor chip via an electrostatic interaction between negative charges of carboxymethyl dextran on the sensor chip and positive charges of the ligand to be immobilized. This step is called "preconcentration of the ligand". To assess the preconcentration of MT-18 on a sensor chip, MT-18 was prepared in buffers with varying pHs (PBS, 10 mM Na-acetate buffer, pH 6.0, 5.0 or 4.0) and injected onto the sensor chip on which no ligand has been immobilized.

### BIACORE assay conditions (preconcentration of MT-18)

**·**Flow rate: 5 µL/min
**·**Test sample: 10 µL (for concentrating MT-18 in a matrix)

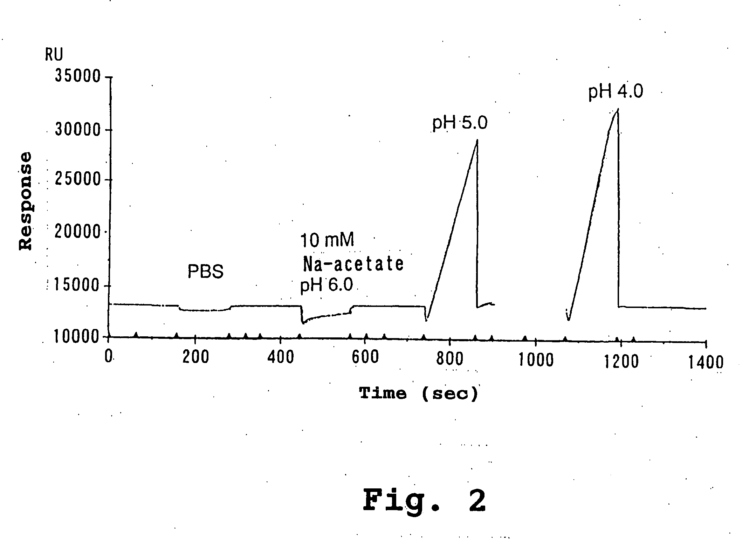

The results are shown in Fig. 2. MT-18 was concentrated in the matrix slightly at pH 6.0, and efficiently at pH 5.0 and 4.0. In view of the, structural stability of the protein, 10 mM Na-acetate buffer, pH 5.0 was chosen as a solution for immobilizing MT-18.

### Example 2: Immobilization of MT-18

MT-18 was prepared at 100 µg/mL in 10 mM Na-acetate buffer, pH 5.0 and immobilized on a sensor chip under the following conditions.

### BIACORE assay conditions (preparation of an MT-18 immobilized chip)

**·**Flow rate: 5 µL/min
**·**Dilute NHS+EDC: 50% (as a 1:1 mixture of NHS and EDC)
**·**NHS+EDC: 35 µL (for activating the matrix)
**·**100 µg/mL MT-18: 50 µL (covalently coupled to the matrix)
·1 M Ethanolamine: 35 µL (for blocking unreacted sites)

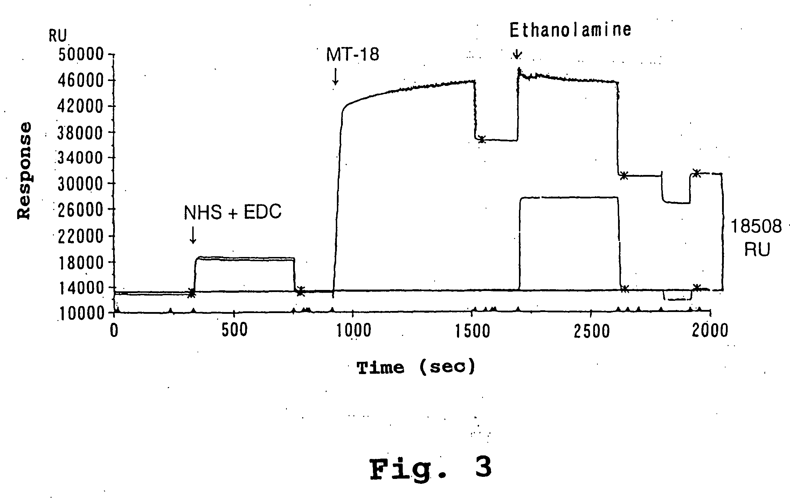

MT-18 was prepared at 100 µg/mL in 10 mM Na-acetate, pH 5.0 and immobilized on a sensor chip by injecting 50 µL after the sensor chip was activated with NHS+EDC. An example of a sensorgram in which MT-18 is immobilized is shown in Fig. 3. As can be seen in Fig. 3, MT-18 corresponding to about 18,000 resonance units (RU) can be immobilized on the sensor chip. This indicates that MT-18 can be immobilized in a sufficient amount for accurate concentration assays under these immobilization conditions.

### Example 3: Verification of the association between soluble human IL-6 receptor and hPM-1 antibody

Soluble human IL-6 receptor (hereinafter referred to as sIL-6R) and hPM-1 antibody were respectively injected onto the MT-18 immobilized chip (using HBS-EP buffer) to detect their interactions.

### BIACORE assay conditions (verification of the association between sIL-6R and hPM-1)

·Flow rate: 5 µL/min
·sIL-6R: 10 µL (to be bound to MT-18)
·10 µg/mL hPM-1: 10 µL (to be bound to sIL-6R)
·10 mM Gly-HCl, pH 2.0: 5 µL (for regeneration)

Separately, a sensor chip activated with EDC+NHS and blocked with ethanolamine but having no ligand immobilized (hereinafter referred to as reference chip) was prepared by the procedure below to show that SIL-6R and hPM-1 are not non-specifically adsorbed to the sensor chip.

### BIACORE assay conditions (preparation of a reference chip)

·Flow rate: 5 µL/min
·Dilute NHS+EDC: 50% (as a 1:1 mixture of NHS and EDC)
·NHS+EDC: 35 µL (for activating the matrix)
·1 M Ethanolamine: 35 µL (for blocking unreacted sites)

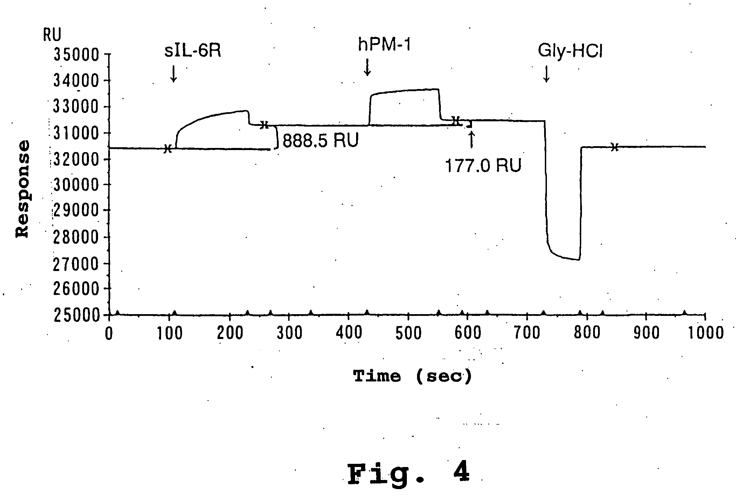

Evaluation was made to demonstrate that sIL-6R binds to MT-18 immobilized on the sensor chip and that hPM-1 binds to sIL-6R. The results are shown in Fig. 4. When sIL-6R was injected onto the MT-18 immobilized chip, a rise in the sensorgram was observed. When hPM-1 was subsequently injected, hPM-1 was shown to bind onto the sensor chip. These results show that sIL-6R can bind to immobilized MT-18 and that hPM-1 can bind to sIL-6R captured on immobilized MT-18.

In the same manner, sIL-6R and hPM-1 were injected on the reference chip to examine their interactions with the sensor chip, showing that the amounts of sIL-6R culture medium and hPM-1 bound to the reference chip were about 40 RU and 30 RU, respectively. As compared with the amounts of sIL-6R and hPM-1 bound to MT-18 (several hundreds of RU, see Fig. 4), non-specific binding to the dextran matrix seemed to be negligible. It also seemed that coexistent proteins in the sIL-6R culture medium have little influence. Here, sIL-6R was used as a cell culture solution containing human IL-6R without any special purification operation. Thus, the sIL-6R sample contains various proteins (e.g. bovine serum albumin) used for culture in addition to IL-6R. These results show that sIL-6R can bind to MT-18 even when these coexistent proteins are contained.

### Example 4: Study on regeneration conditions

The following acid solutions with varying pHs were tested for the suitability as regeneration solutions for dissociating sIL-6R bound to the MT-18 immobilized chip and hPM-1.
**·**10 mM Gly-HCl, pH 2.0, 2.5
·0.15 M Na-citrate, pH 5.0, 4.0, 3.0

Na-citrate, pH 5.0 and 4.0 showed a low capacity to dissociate sIL-6R from MT-18. Na-citrate, pH 3.0 and Gly-HCl, pH 2.5 could dissociate sIL-6R bound to MT-18. Gly-HCl, pH 2.0 was found to be unsuitable as a regeneration solution because it greatly reduced the amount of bound sIL-6R while the base line rose modestly. The remaining Gly-HCl, pH 2.5 and Na-citrate, pH 3.0 were compared to show that Gly-HCl, pH 2.5 caused a small variation in base line while Na-citrate, pH 3.0 caused a small variation in the amount of bound sIL-6R. Gly-HCl, pH 2.5 showing a small variation in base line was chosen as a regeneration solution because the performance of the sensor chip seems to be degraded by incomplete dissociation of the analyte from the ligand if regeneration is insufficient.

On the basis of the above assessment, 10 mM Gly-HCl, pH 2.5 was chosen as a regeneration solution. Then, regeneration conditions such as flow rate and injection amount were tested. It is empirically known from previous experiments that components undissociated from the sensor chip increase with the number of times the sensor chip is used even if a regeneration solution is used. It is also shown that the sensor chip cannot be sufficiently washed unless a regeneration solution is injected twice when 5 µL of the regeneration solution is injected at a flow rate of 5 µL/min.

Thus, regeneration conditions involved 2 x 5 µL at 5 µL/min or 1 x 10 µL at 10 µL/min. As a result, it was shown that the variation of the binding activity of hPM-1 to sIL-6R was smaller when 10 µL was injected at a flow rate of 10 µL/min. Thus, regeneration conditions were chosen to involve 1 x 10 µL at 10 µL/min.

It is also empirically known that the amount of bound hPM-1 decreases with the increase of the number of regeneration cycles. This tendency was also found when another protein (IL-5) was immobilized on the sensor chip (J. Immunol. Methods, 1997, 1-15). According to the test method described, the number of measurements of samples is limited to satisfy the criterion that "the variation in the amount of bound analyte should be within 20%". In the present assay system, it is also difficult to completely avoid the decrease of the amount of bound hPM-1, and the number of measurements should be limited to satisfy some criterion suitable for the purpose. Thus, the measurement was repeated at N=30 under the regeneration conditions described above in order to determine the limit number of repeated measurements. A criterion was established so that the variation in the amount of bound hPM-1 should be within 5% in CV. As a result, it was shown that the number of measurements satisfying the intended criterion was about 20. This led to the limitation that "the number of measurements of samples should be about 20 using one MT-18 immobilized sensor chip".

### Example 5: Comparison of SPR- and ELISA-based assays

The SPR-based assay as defined in the present study was compared with the current quality test method based on ELISA for evaluating the biological activity of hPM-1. The standard hPM-1. sample and test samples (untreated and accelerated at 60°C for 2 weeks) used in the assays are shown below.
·Standard sample: hPM-1 Lot No. 96D01, 4.48 mg/mL (UV)
·Test samples: hPM-1 Lot No. 99L01, Initial, 58.1 mg/mL (UV) hPM-1 Lot No. 99L01, 60°C-2W

### (1) SPR-based assay

According to the procedure described in Example 2, MT-18 was immobilized on a sensor chip. Preparation procedures of the standard sample and test samples and SPR-based assay conditions are shown below.

### Preparation procedures of samples

1. The hPM-1 standard and test samples were prepared at 50 µg/mL in HBS-EP buffer. (Each test sample was prepared at N=3.)
2. The hPM-1 stock at 50 µg/mL was respectively diluted in HBS-EP buffer to 1, 2, 5, 10 and 20 µg/mL for the standard sample and to 5 µg/mL for the test samples using BIACORE 2000.
3. Binding activity to IL-6R was measured in the diluted solution at N=2.

### BIACORE assay conditions

·Flow rate: 10 µL/min
·sIL-6R: 20 µL (to be bound to MT-18)
·hPM-1: 40 µL (to be bound to sIL-6R)
·10 mM Gly-HCl, pH 2.5: 10 µL (for regeneration)

### Data analysis

Using BIACORE analytical software, BIAevaluation Ver. 3.0, a calibration curve was generated from the amount of bound hPM-1 in the standard sample. For fitting to the calibration curve, the 4-parameter fit algorithm available from the analytical software was used. The amounts of bound hPM-1 in the test samples were converted into hPM-1 concentrations using the calibration curve as generated. The average of the hPM-1 concentrations in the test samples at N=2 was taken as measured value.

### (2) ELISA-based assay

To the MT-18-immobilized plate was bound sIL-6R. The hPM-1 antibody was added to this plate and bound to sIL-6R on the plate. After unreacted hPM-1 antibody was washed off, enzyme-labeled anti-human IgG antibody was added. After the plate was washed, enzymatic activity was assayed to calculate the amount of the hPM-1 antibody bound to IL-6R on the basis of the enzymatic activity of the standard solution.

### (3) Comparison between SPR- and ELISA-based assays

The evaluation results of the antigen-binding activity of accelerated samples of the hPM-1 stock by SPR and ELISA are shown in Tables 1 and 2.

**Table 1**

| Results of SPR-based assay for binding activity to IL-6 receptor | | | | |
|---|---|---|---|---|
| Sample | hPM-1 (mg/mL) | Average | CV (%) | % Initial |
| | 51.81 | | | |
| hPM-1 99L01 Initial | 50.63 | 49.68 | 5.50 | - |
| | 46.60 | | | |
| | 43.17 | | | |
| hPM-1 99L01 60-2W | 39.68 | 40.67 | 5.35 | 81.9 |
| | 39.17 | | | |

**Table 2**

| Results of ELISA-based assay for binding activity to IL-6 receptor | | | | |
|---|---|---|---|---|
| Sample | hPM-1 (mg/mL) | Average | CV (%) | % Initial |
| | 90.08 | | | |
| hPM-1 99L01 Initial | 58.70 | 70.47 | 24.26 | - |
| | 62.64 | | | |
| | 56.22 | | | |
| hPM-1 99L01 60-2W | 37.66 | 48.41 | 19.88 | 68.7 |
| | 51.36 | | | |

The binding activities of hPM-1 99L01 to IL-6R at the initial stage and after 2 weeks at 60°C were converted into hPM-1 concentrations which were calculated at 49.7 mg/mL and 40.7 mg/mL in SPR, and 70.5 mg/mL and 48.5 mg/mL in ELISA. The residual rate of the binding activity after 2 weeks at 60°C relative to that at the initial stage was 81.9% (SPR) and 68.7% (ELISA).

These results demonstrated that the acceleration-induced decrease of the activity of the hPM-1 stock could be detected by both SPR and ELISA.

The precision of each assay for the binding activity to antigen was 5.5% in SPR or 24.3% in ELISA expressed in CV. This result showed that the SPR-based assay is a method for evaluating the binding activity of hPM-1 to IL-6R with high precision.

## Claims

1. A method for assaying via surface plasmon resonance the binding activity of a first ligand to a ligand-binding protein which is chemically less stable so that it cannot be reused on a sensor chip surface because of its deactivation by washing comprising:
immobilizing a second ligand that binds to the ligand-binding protein at a binding site distinct from the binding site of the first ligand to the ligand-binding protein;
binding the protein to the second ligand; and
then measuring the binding activity of the first ligand to the protein.

2. The method of claim 1 wherein the chemically less stable ligand-binding protein is an antigenic protein.

3. The method of claim 1 wherein the chemically less stable ligand-binding protein is a soluble receptor.

4. The method of claim 3 wherein the soluble receptor is a cytokine receptor.

5. The method of claim 4 wherein the cytokine receptor is an IL-6 receptor.

6. The method of any one of claims 1 to 5 wherein the first ligand is an antibody.

7. The method of claim 6 wherein the antibody is an anti-IL-6 receptor antibody.

8. The method of claim 6 or 7 wherein the antibody is a humanized antibody.

9. The method of any one of claims 1 to 8 wherein the second ligand is an antibody.

10. A method for assaying via surface plasmon resonance the biological activity of a first ligand that binds to a ligand-binding protein which is chemically less stable so that it cannot be reused on a sensor chip surface because of its deactivation by washing, comprising:
immobilizing a second ligand that binds to the ligand-binding protein at a binding site distinct from the binding site of the first ligand to the ligand-binding protein;
binding the protein to the second ligand; and
then determining the biological activity of the first ligand by measuring the binding activity of the first ligand to the protein.

## Patentansprüche

1. Verfahren zum Testen der Bindungsaktivität eines ersten Liganden an ein Liganden-Bindungsprotein, das chemisch weniger stabil ist, wodurch es wegen Inaktivierung durch Waschen nicht auf einer Sensorchip-Oberfläche wiederverwendet werden kann, mittels Oberflächenplasmonenresonanz, umfassend:
das Immobilisieren eines zweiten Liganden, der an das Liganden-Bindungsprotein an einer Bindestelle bindet, die sich von der Bindestelle des ersten Liganden an das Liganden-Bindungsprotein unterscheidet;
das Binden des Proteins an den zweiten Liganden; und
dann das Messen der Bindungsaktivität des ersten Liganden an das Protein.

2. Verfahren nach Anspruch 1, wobei das chemisch weniger stabile Liganden-Bindungsprotein ein antigenes Protein ist.

3. Verfahren nach Anspruch 1, wobei das chemisch weniger stabile Liganden-Bindungsprotein ein löslicher Rezeptor ist.

4. Verfahren nach Anspruch 3, wobei der lösliche Rezeptor ein Cytokinrezeptor ist.

5. Verfahren nach Anspruch 4, wobei der Cytokinrezeptor ein IL-6 Rezeptor ist.

6. Verfahren nach einem der Ansprüche 1 bis 5, wobei der erste Ligand ein Antikörper ist.

7. Verfahren nach Anspruch 6, wobei der Antikörper ein Anti-IL-6-Rezeptor-Antikörper ist.

8. Verfahren nach Anspruch 6 oder 7, wobei der Antikörper ein humanisierter Antikörper ist.

9. Verfahren nach einem der Ansprüche 1 bis 8, wobei der zweite Ligand ein Antikörper ist.

10. Verfahren zum Testen der biologischen Aktivität eines ersten Liganden, der an ein Liganden-Bindungsprotein bindet, das chemisch weniger stabil ist, wodurch es wegen Inaktivierung durch Waschen nicht auf einer Sensorchip-Oberfläche wiederverwendet werden kann, mittels Oberflächenplasmonenresonanz, umfassend:
das Immobilisieren eines zweiten Liganden, der das Liganden-Bindungsprotein an einer Bindestelle bindet, die sich von der Bindestelle des ersten Liganden an das Liganden-Bindungsprotein unterscheidet;
das Binden des Proteins an den zweiten Liganden; und
dann das Bestimmen der biologischen Aktivität des ersten Liganden durch Messen der Bindungsaktivität des ersten Liganden an das Protein.

## Revendications

1. Procédé pour tester par résonance plasmon de surface l'activité de liaison d'un premier ligand à une protéine liant un ligand, et qui est chimiquement moins stable de telle sorte qu'elle ne peut être réutilisée sur une surface de puce de capteurs en raison de sa désactivation par lavage, comprenant :
l'immobilisation d'un second ligand qui se lie à la protéine liant un ligand en un site de liaison distinct du site de liaison du premier ligand sur la protéine liant un ligand;
la liaison de la protéine au second ligand ; et
ensuite, la mesure de l'activité de liaison du premier ligand à la protéine.

2. Procédé selon la revendication 1, dans lequel la protéine chimiquement moins stable liant un ligand est une protéine antigénique.

3. Procédé selon la revendication 1, dans lequel la protéine chimiquement moins stable liant un ligand est un récepteur soluble.

4. Procédé selon la revendication 3, dans lequel le récepteur soluble est un récepteur de cytokine.

5. Procédé selon la revendication 4, dans lequel le récepteur de cytokine est un récepteur d'IL-6.

6. Procédé de l'une quelconque des revendications 1 à 5, dans lequel le premier ligand est un anticorps.

7. Procédé selon la revendication 6, dans lequel l'anticorps est un anticorps récepteur d'anti-IL-6.

8. Procédé selon la revendication 6 ou 7, dans lequel l'anticorps est un anticorps humanisé.

9. Procédé selon l'une quelconque des revendications 1 à 8, dans lequel le second ligand est un anticorps.

10. Procédé pour tester par résonance plasmon de surface l'activité biologique d'un premier ligand qui se lie à une protéine liant un ligand et qui est chimiquement moins stable de telle sorte qu'elle ne peut être réutilisée sur une surface de puce de capteur en raison de sa désactivation par lavage, comprenant:
l'immobilisation d'un second ligand qui se lie à la protéine liant un ligand en un site de liaison distinct du site de liaison du premier ligand sur la protéine liant un ligand;
la liaison de la protéine au second ligand ; et
ensuite, la détermination de l'activité biologique du premier ligand par mesure de l'activité de liaison du premier ligand à la protéine.
